# EUROPEAN PATENT SPECIFICATION

(11) **EP 2 151 261 B1**
(45) Date of publication and mention of the grant of the patent: **09.01.2013**
(21) Application number: 08764686.5
(22) Date of filing: 26.05.2008
(51) Int. Cl.: A61B 18/20

(54) **OPTICAL DEPILATION APPARATUS**
OPTISCHES DEPILATIONSGERÄT
APPAREIL D'EPILATION OPTIQUE

(30) Priority: 28.05.2007 JP 2007141099
(43) Date of publication of application: 10.02.2010
(73) Proprietor: Panasonic Corporation, Osaka 571-8501 (JP)
(72) Inventor: FUJIKAWA, Shoji, Kadoma-shi Osaka (JP)
(74) Representative: Müller-Boré & Partner Patentanwälte
(86) International application number: PCT/JP2008/059653
(87) International publication number: WO 2008/146788

(56) References cited:
- EP-A1- 1 262 210
- WO-A1-2004/043543
- JP-A- 2002 030 620
- JP-B2- 3 577 439
- JP-B2- 61 030 590
- JP-U- 49 146 793

## Description

### Technical Field

The present invention relates to an optical depilation apparatus that performs depilation by irradiating skin with light from a light irradiation unit.

### Background Art

An optical depilation apparatus in which hair papilla is heated by transferring light energy emitted by a laser diode or a flashlamp to the hair, thereby making it difficult for nutrients to reach the hair, is known as a device for conducting depilation. In such an optical depilation apparatus, the skin with hairs is required to be irradiated with light from the direction perpendicular to the skin. Accordingly, in the optical depilation apparatus disclosed in Patent Document 1 or Patent Document 2 below, a plurality of contact sensors are disposed in positions surrounding the laser beam irradiation portion of the distal end of the apparatus, the optical depilation apparatus is determined to have a perpendicular posture when the contact sensors detect contact with the skin, and the laser beam irradiation is then started.

However, in the above-described optical depilation apparatus, safety measures taken to prevent the light from leaking to the outside are insufficient. In a case where a flashlamp generating flashes is used as the light source, the flashes easily leak to the outside and can enter the eyes of the user or other people. Furthermore, the flashes can be unintentionally and erroneously emitted when the optical depilation apparatus is maintained and the flashes can enter the eyes of the user or other people.
Therefore, more reliable safety measures are needed.
Patent Document 1: Japanese Unexamined Patent Publication No. 2005-278724.
Patent Document 2: Japanese Unexamined Patent Publication No. 2006-525036.
Further, the European patent application EP 1 262 210 A1 discloses a laser beam radiation projector. A laser beam throwing aperture is made in the tip of the head of the device to be applied to the skin. The head has a heat sink provided therein, and the heat sink has a through hole made at its center. A CCD camera is placed at the end of the through hole, and is directed to the laser beam throwing aperture. A semiconductor laser source is so attached to the heat sink that it may project a laser beam at an oblique angle relative to the center line of the head. A condenser lens is placed ahead of the semiconductor laser source to converge the laser beam to the laser beam throwing aperture. An illumination source is attached to the heat sink to illuminate the laser beam throwing aperture.

### Disclosure of the Invention

It is an object of the present invention to provide an optical depilation apparatus that resolves the above-described problems.

It is another object of the present invention to provide an optical depilation apparatus of high safety, in which the light irradiating the skin during the depilation treatment can be prevented from leaking and entering the eyes, maintenance can be facilitated, and the light for depilation can be prevented from being emitted at an unintended time and entering the eyes.

In order to attain the above-described objects, the optical depilation apparatus according to the present invention is an optical depilation apparatus for performing depilation by irradiating skin with light, comprising: an apparatus body; a light source unit that is provided at the apparatus body and capable of emitting light for irradiating skin; a light shielding pipe that has a skin contact surface at an end surface on a side opposite to the apparatus body in an axial direction of the light shielding pipe and is attached detachably to the apparatus body so as to surround a portion of the apparatus body which radiates light emitted from the light source unit; and a control circuit that disables the emission of light from the light source unit in a state in which the light shielding pipe is not attached to the apparatus body,
wherein:
the light shielding pipe is free to move back and forth in the axial direction;
the optical depilation apparatus further comprises a spring member that biases the light shielding pipe in the axial direction to the side opposite to the apparatus body; a plurality of contact sensors that are provided with a predetermined spacing in a circumferential direction at the skin contact surface; and an irradiation switch that is provided at the apparatus body; and
the control circuit has a first switch, a second switch and a third switch, wherein the first switch is switched OFF when the light shielding pipe is not attached to the apparatus body and when the depression amount of the light shielding pipe in the axial direction with respect to the apparatus body is less than a set depression amount that has been set in advance even when the light shielding pipe is attached to the apparatus body, wherein the first switch is ON when the light shielding pipe is attached to the apparatus body and the depression amount of the light shielding pipe with respect to the apparatus body is equal to or greater than the set depression amount, wherein the second switch is turned ON when all the contact sensors detect contact with the skin, wherein the third switch is turned ON when a user pushes the irradiation switch, wherein the control circuit interrupts power supply to the light source unit so as to disable the emission of light from the light source unit when the first switch, the second switch or the third switch is OFF, wherein the control circuit supplies power to the light source unit when the first switch, the second switch and the third switch are all turned ON, and wherein an operation port is formed at the light shielding pipe so as to expose the irradiation switch when the depression amount of the light shielding pipe with respect to the apparatus body is equal to or greater than the set depression amount.

According to another aspect of the present invention, an optical depilation apparatus for performing depilation by irradiating skin with light comprises:
an apparatus body; a light source unit that is provided at the apparatus body and capable of emitting light for irradiating skin; a light shielding pipe that has a skin contact surface at an end surface on a side opposite to the apparatus body in an axial direction of the light shielding pipe and is attached detachably to the apparatus body so as to surround a portion of the apparatus body which radiates light emitted from the light source unit; and a control circuit that disables the emission of light from the light source unit in a state in which the light shielding pipe is not attached to the apparatus body,
wherein:
the light shielding pipe is free to move back and forth in the axial direction;
the optical depilation apparatus further comprises a spring member that biases the light shielding pipe in the axial direction to the side opposite to the apparatus body; and an irradiation switch that is provided at the apparatus body,
wherein the control circuit has a first switch and a third switch, wherein the first switch is switched OFF when the light shielding pipe is not attached to the apparatus body and when the depression amount of the light shielding pipe in the axial direction with respect to the apparatus body is less than a set depression amount that has been set in advance even when the light shielding pipe is attached to the apparatus body, wherein the first switch is ON when the light shielding pipe is attached to the apparatus body and a depression amount of the light shielding pipe with respect to the apparatus body is equal to or greater than the set depression amount, wherein the third switch is turned ON when a user pushes the irradiation switch, wherein the control circuit interrupts power supply to the light source unit so as to disable the emission of light from the light source unit when the first switch or the third switch is turned OFF, wherein the control circuit supplies power to the light source unit when the first switch is turned ON and the third switch is turned ON, and wherein an operation port is formed at the light shielding pipe so as to expose the irradiation switch when the depression amount of the light shielding pipe with respect to the apparatus body is equal to or greater than the set depression amount.

### Brief Description of the Drawings

[Fig. 1] Fig. 1 is a cross-sectional view illustrating schematically a state in which a light shielding pipe of the optical depilation apparatus of one embodiment of the invention is in the uppermost position.
[Fig. 2] Fig. 2 is a cross-sectional view illustrating schematically a state in which the light shielding pipe of the optical depilation apparatus of one embodiment of the invention is in the downmost position.
[Fig. 3] Fig. 3 is a front view of the optical depilation apparatus shown in Fig. 1.
[Fig. 4] Fig. 4 is a side view of the optical depilation apparatus shown in Fig. 1.
[Fig. 5] Fig. 5 is a plan view of the optical depilation apparatus shown in Fig. 1.
[Fig. 6] Fig. 6 is a front view of the optical depilation apparatus illustrating a state in which the light shielding pipe in the optical depilation apparatus shown in Fig. 1 is depressed down and an irradiation switch is exposed.
[Fig. 7] Fig. 7 is a side view of the optical depilation apparatus illustrating a state in which the light shielding pipe in the optical depilation apparatus shown in Fig. 1 is depressed down and the irradiation switch is exposed.
[Fig. 8] Fig. 8 is a cross-sectional view illustrating the structure of the irradiation switch.
[Fig. 9] Fig. 9 is a side view illustrating the structure of the irradiation switch.
[Fig. 10] Fig. 10 is a circuit block diagram illustrating schematically an example of control circuit.
[Fig. 11] Fig. 11 is a circuit block diagram illustrating schematically another example of control circuit.
[Fig. 12] Fig. 12 is a circuit block diagram illustrating schematically yet another example of control circuit.

### Best Mode for Carrying Out the Invention

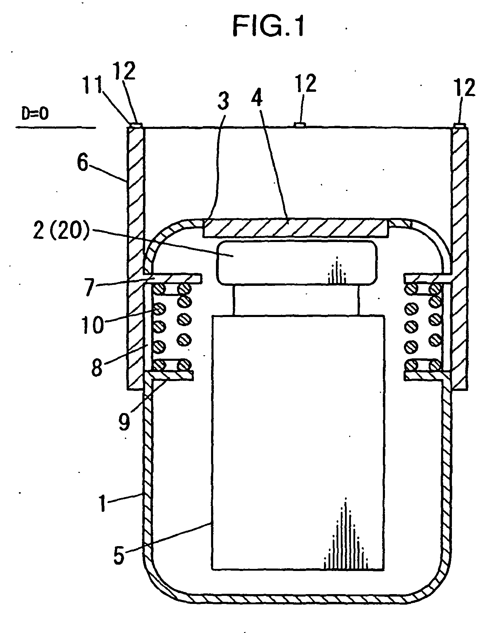

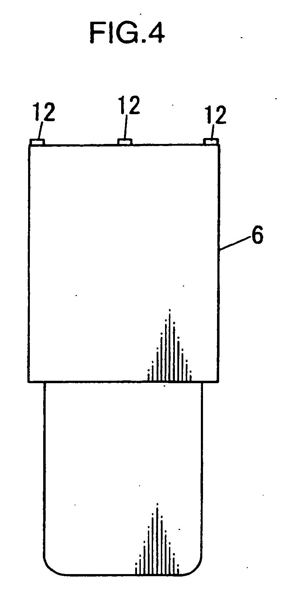

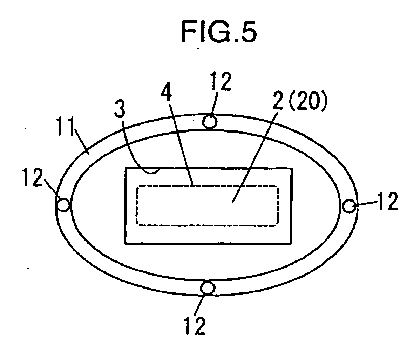

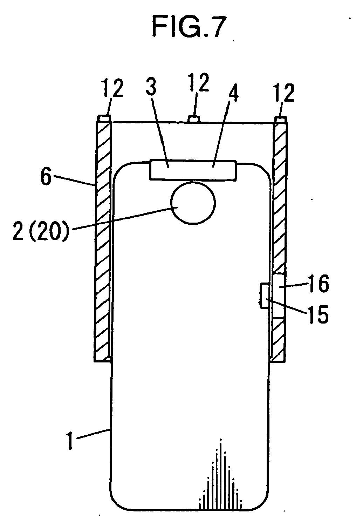

The embodiments of the present invention will be described below with reference to the appended drawings. Figs. 1 to 7 illustrate schematically the entire structure of the optical depilation apparatus according to one embodiment of the invention.

The optical depilation apparatus of the present embodiment is provided with a box-shaped apparatus body 1 that can be grasped by one hand. A flashlight 2 that is used as a light source unit 20 is accommodated in the apparatus body 1. The flashlight 2 is composed of a xenon lamp. A light irradiation port 3 that links the inside of the apparatus body 1 with the outside is opened in a position close to the flashlight 2 at the distal end of the apparatus body 1. This light irradiation port 3 is disposed so as to be opposite the flashlight 2. A transparent lamp cover 4 is fitted in the light irradiation port 3. The lamp cover 4 is provided at the apparatus body 1 so that the outer surface of the lamp cover 4 is exposed. The flashlight 2 emits pulse-shaped flashes and these flashes are transmitted through the lamp cover 4 and irradiated to the outside of the apparatus body 1. Thus, the light emitted by the flashlight 2 is emitted from the apparatus body 1 at the lamp cover 4.

The optical depilation apparatus is also provided with a light shielding pipe 6 detachably attached in a location around the light irradiation port 3 on the outer circumference of the apparatus body 1. The light shielding pipe 6 is formed as an elliptical pipe by using a flexible elastic material such as rubber or elastomer. Further, the light shielding pipe 6 is floatably attached to the apparatus 1. The "floatable state" as referred to herein means a state in which the light shielding pipe 6 is free to move back and forth in the axial direction, while the light shielding pipe 6 is being biased in the axial direction to the side opposite to the apparatus body 1. The direction on the side opposite to the apparatus body 1 in the axial direction of the light shielding pipe 6, that is, the direction forward along the light irradiation direction will be referred to hereinbelow as "upward", and the direction on the side the apparatus body 1 in the axial direction of the light shielding pipe 6, that is, the direction rearward along the light irradiation direction will be referred to hereinbelow as "downward".

In the example shown in the figure, a mechanism composed of a linking protruding portion 7, a linking port 8, a receiving portion 9, and a spring member 10 is provided as an attachment mechanism for floatably attaching the light shielding pipe 6 to the apparatus body 1. The linking protruding portion 7 extends inward from the inner circumferential surface of the light shielding pipe 6. The linking port 8 is opened at the side wall of the apparatus body 1 and serves to receive movably in the vertical direction the linking protruding portion 7 and couple the light shielding pipe 6 to the apparatus body 1 so that the light shielding pipe can move back and forth in the vertical direction. The receiving portion 9 is provided at the inner circumferential surface of the apparatus body 1 in a position adjacent to the lower end edge of the linking port 8. The spring member 10 is composed of a compression coil spring inserted between the receiving portion 9 and linking protruding portion 7. The attachment of the light shielding pipe 6 to and detachment of the light shielding pipe 6 from the apparatus body 1 are conducted, for example, by pulling out the apparatus body 1 from the light shielding pipe 6 and inserting the apparatus body 1 into the light shielding pipe 6, while causing certain elastic deformation of the linking protruding portion 7 of the light shielding pipe 6.

The light shielding pipe 6 has a skin contact surface 11 as shown in Figs. 1 to 5. The skin contact surface 11 is pushed into direct contact with a skin S of a human body and composed of an end surface on the side opposite to the apparatus body 1 in the axial direction of the light shielding pipe 6, that is, of an upper end surface of the light shielding pipe 6. A plurality of contact sensors 12 are provided with an equal spacing in the circumferential direction on the skin contact surface 11. A control circuit 5 determines that the skin S of the human body is in intimate contact with the distal end portion of the light shielding pipe 6 and enables the emission of light from the flashlight 2 only in a case where all the contact sensors 12 detect contact with the skin S.

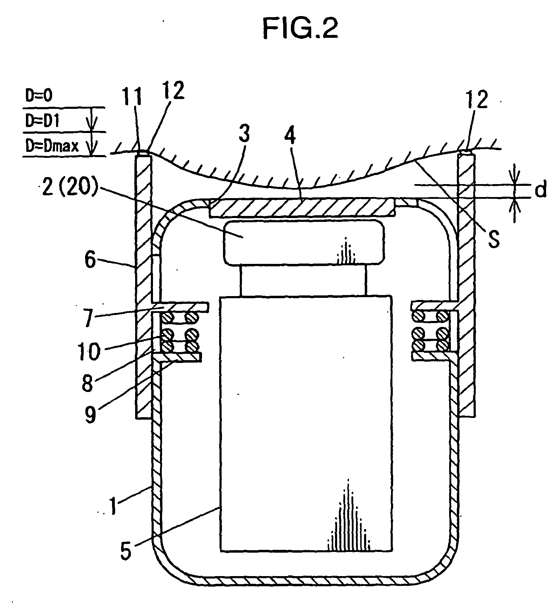

Fig. 1 illustrates a state in which the light shielding pipe 6 is in the uppermost position in the optical depilation apparatus, that is, a state in which a depression amount of the light shielding pipe 6 with respect to the apparatus body 1 is zero (D = 0). Fig. 2 illustrates a state in which the light shielding pipe 6 is in the lowermost position in the optical depilation apparatus, that is, a state in which the depression amount D is equal to the maximum depression amount Dmax. The vertical size of the light shielding pipe 6 is set such that the skin contact surface 11 is positioned above the light irradiation port 3 of the apparatus body 1 in both states. Further, the vertical size of the light shielding pipe 6 is set such that a gap d is ensured between the light irradiation port 3 and the skin S that bulges downward by the pressing force of the skin contact surface 11 even in a state in which the light shielding pipe 6 has been depressed to the maximum depression amount Dmax as shown in Fig. 2.

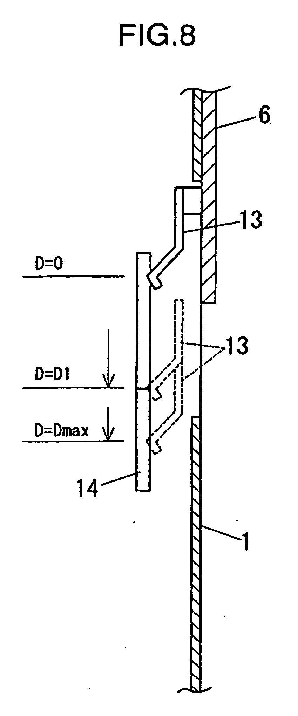

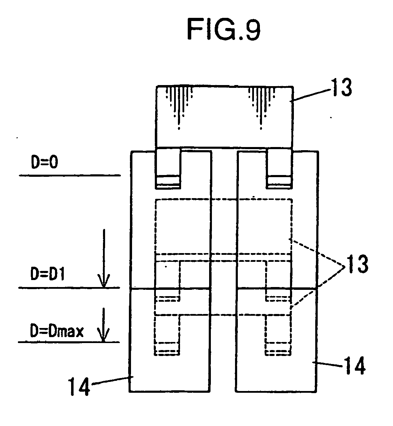

As shown in Figs. 8 and 9, a switch spring 13 is fixed to the inner circumferential surface of the light shielding pipe 6, and a pair of connection terminals 14 are incorporated in the apparatus body 1. The switch spring 13 comes into contact with the pair of connection terminals 14 of the apparatus body 1 and enables electric conduction with the connection terminals 14 only in a case where the downward depression amount D of the light shielding pipe 6 with respect to the apparatus body 1 is equal to or greater than a set depression amount D1 that has been set in advance (0 < D1 < Dmax). Where it is possible to conduct electricity between the switch spring 13 and connection terminals 14, the control circuit 5 enables light emission from the flashlight 2. Light emission from the flashlight 2 is enabled by the control circuit 5 on condition that the switch spring 13 and the connection terminals 14 are in contact. Therefore, the flashlight 2 can emit light only in a case where the light shielding pipe 6 is attached to the apparatus body 1 in a predetermined position, and the flashlight 2 is unable to emit light in a state where the light shielding pipe 6 is detached from the apparatus body 1.

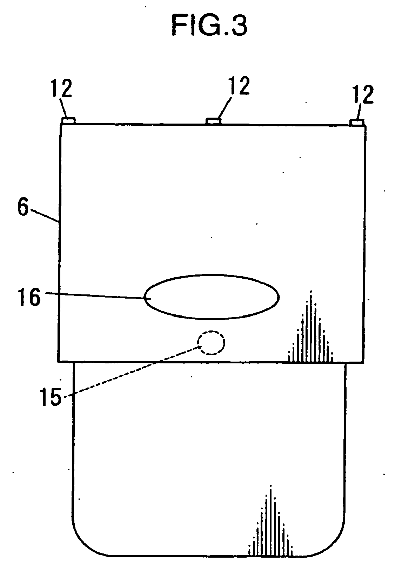

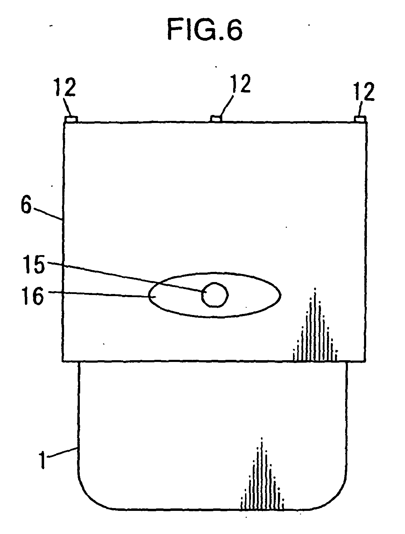

As shown in Figs. 3 and 6, an irradiation switch 15 is provided in the center of the front side of the outer circumferential surface of the apparatus body 1. The irradiation switch 15 enables the user to ON/OF operate manually the emission of light from the flashlight 2. An operation port 16 is formed at the light shielding pipe 6 so as to expose the irradiation switch 15 when the light shielding pipe 6 is depressed downward to a certain amount. The operation port 16 is provided in a position such that the irradiation switch 15 can be exposed when the switch spring 13 and the connection terminals 14 come into contact. Further, the operation port 16 is formed to have a size such that makes it possible to expose the irradiation switch 15 at least when the depression amount D of the light shielding pipe 6 is within a range of D1 to Dmax. The control circuit 5 enables light emission from the flashlight 2 only in a case where the user continuously pushes the irradiation switch 15 exposed from the operation port 16 or where the irradiation switch is fixed in a pushed-down state.

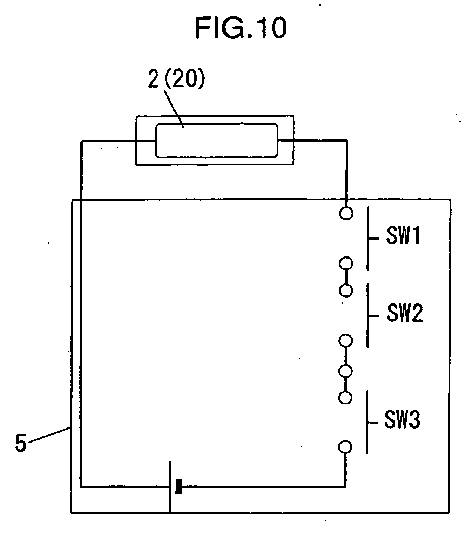

As shown schematically in Fig. 10, the control circuit 5 of the present embodiment is provided with a triple switch circuit. The triple switch circuit is configured by connecting a first switch SW1, a second switch SW2, and a third switch SW3 in series.

The first switch SW1 is configured with the switch spring 13 and the connection terminals 14 (see Figs. 8 and 9). The first switch SW1 is switched ON when the depression amount D of the light shielding pipe 6 with respect to the apparatus body 1 is equal to or greater than the set depression amount D1 in a state in which the light shielding pipe 6 is attached to the apparatus body 1, but switched OFF when the light shielding pipe 6 is not attached to the apparatus body 1 and when the depression amount of the light shielding pipe 6 with respect to the apparatus body 1 is less than the set depression amount D1 even when the light shielding pipe 6 is attached to the apparatus body 1.

The second switch SW2 is electrically connected to each contact sensor 12 so as to be switched ON when all the contact sensors 12 provided at the distal end of the light shielding pipe 6 detect contact with the skin S. The second switch SW2 is OFF in a case where at least one of a plurality of contact sensors 12 does not detect contact with the skin S.

The third switch SW3 is ON when the user pushes the irradiation switch 15 disposed at the apparatus body 1 and OFF when the irradiation switch 15 is not pushed.

The control circuit 5 supplies power to the flashlight 2 and causes the flashlight 2 to emit pulsed light only in a case where the following conditions are satisfied: all the switches SW1, SW2, SW3 are ON, that is, the depression amount D of the light shielding pipe 6 with respect to the apparatus body 1 is equal to or greater than the set depression amount D1 in a state in which the light shielding pipe 6 is attached to the apparatus body 1, the distal end of the light shielding pipe 6 is in intimate contact with the skin S and all the contact sensors 12 are ON, and the user pushes down the irradiation switch 15 of the apparatus body 1. In other words, in a case where the light shielding pipe 6 is not attached to the apparatus body 1 and where the depression amount of the light shielding pipe 6 with respect to the apparatus body 1 is less than the set depression amount D1 even when the light shielding pipe 6 is attached to the apparatus body 1, the first switch SW1 is OFF and, therefore, the control circuit 5 interrupts power supply to the flashlight 2, thereby disabling light emission from the flashlight 2. Further, in a case where at least one contact sensor 12 provided at the skin contact surface 11 of the light shielding pipe 6 does not detect contact with the skin S, the second switch SW2 is OFF and, therefore, the control circuit 5 interrupts power supply to the flashlight 2, thereby disabling light emission from the flashlight 2. In a case where the irradiation switch 15 is not pushed, the third switch SW3 is OFF and, therefore, the control circuit 5 interrupts power supply to the flashlight 2, thereby disabling light emission from the flashlight 2.

When the optical depilation apparatus of the present invention having the above-described configuration is used by the user, the user grasps the apparatus body 1 with one hand, brings the skin contact surface 11 of the light shielding pipe 6 into contact with the skin S, and positions the skin S in the treatment object zone inside the region surrounded by the ring-shaped skin contact surface 11. In this state, the user further pushes the apparatus body 1 toward the skin S. As a result, the light shielding pipe 6 is pushed down against the biasing force of the spring member 10, and the biasing force applied from the spring member 10 upward to the light shielding pipe 6 increases accordingly. Due to the biasing force of the spring member 10, the skin contact surface 11 of the light shielding pipe 6 is brought into intimate contact with the skin S over the entire periphery. In this state, even if the user somewhat moves the apparatus body 1, the light shielding pipe 6 that is in the floating state maintains the state of intimate contact with the skin S, while following the movement of the apparatus body 1 and moving up and down.

When the user then manually switches ON the irradiation switch 15 exposed from the operation port 16 of the light shielding pipe 6 in a state in which the depression amount D of the light shielding pipe 6 is equal to or greater than the set depression amount D1 and every contact sensor 12 detects contact with the skin S, the flashlight 2 emits pulsed light, the light passes through the light irradiation port 3, that is, through the lamp cover 4, and the skin S in the treatment object zone is irradiated thereby. The depilation treatment of the skin S in the treatment object zone is conduced by such irradiation. In this case, because the skin contact surface 11 of the light shielding pipe 6 maintains the state of pressure contact with the skin S over the entire periphery of the skin contact surface 11, the flash irradiated through the light irradiation port 3 is prevented from leaking to the outside, and the flash cannot enter the eyes of the user or other people. Further, because the light shielding pipe 6 is formed from a flexible elastic material such as rubber, the state of intimate contact of the skin contact surface 11 of the light shielding pipe 6 against the skin S can be ensured more effectively.

Further, because the switch for ON/OFF switching the flashlight 2 is the triple switch of the above-described configuration, unless the user intentionally operates the irradiation switch 15 in a state in which the skin contact surface 11 of the light shielding pipe 6 is brought into intimate contact with the skin S by a sufficient pressure force over the entire periphery, the emission of light from the flashlight 2 is disabled and safety is further increased.

Further, because the light shielding pipe 6 is detachably attached to the apparatus body 1, when maintenance is conducted, for example, when the lamp cover 4 is removed and the flashlight 2 is replaced, the light shielding pipe 6 can be detached from the apparatus body 1 to facilitate the maintenance. In addition, in the present embodiment, the emission of light from flashlight 2 is enabled only in a case where the switch spring 13 of the light shielding pipe 6 and the connection terminals 14 of the apparatus body 1 are in contact, that is, when the light shielding pipe 6 is attached to the apparatus body 1. Therefore, as described above, light irradiation can be prevented in a state in which the light shielding pipe 6 is detached from the apparatus body 1, and flashes of the flashlight 2 can be prevented from entering the eyes.

All the features of the embodiment disclosed above are exemplary and should not be construed as limiting. The scope of the present invention is represented by the claims, rather than limited to the above-described embodiment, and includes all the variations that are equivalent to the claims in meaning and scope.

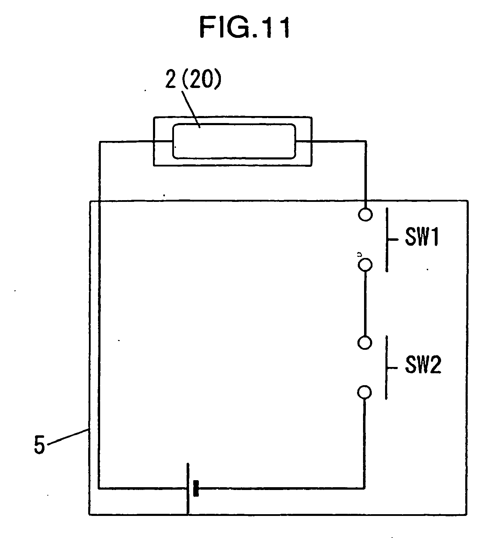

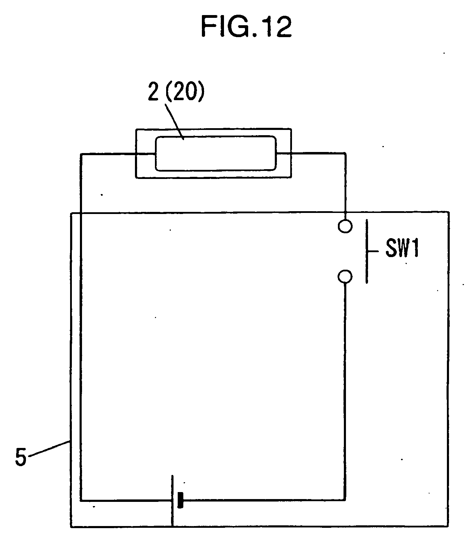

For example, the switch circuit in the control circuit 5 may be a double switch circuit in which the first switch SW1 and second switch SW2 are connected in series, as shown in Fig. 11, or a switch circuit composed only of the first switch SW1, as shown in Fig. 12, rather than the triple switch circuit. In the case where the double switch circuit shown in Fig. 11 is provided in the control circuit 5, power is supplied to the flashlight 2 and the flashlight 2 emits pulsed light at a stage at which the depression amount D of the light shielding pipe 6 with respect to the apparatus body 1 becomes equal to or greater than the set depression amount D1 in a state in which the light shielding pipe 6 is attached to the apparatus body 1, and all the contact sensors 12 are ON due to the intimate contact of the distal end of the light shielding pipe 6 with the skin S. Further, in a case where the switch circuit shown in Fig. 12 is provided in the control circuit 5, power is supplied to the flashlight 2 and the flashlight 2 emits pulsed light at a stage at which the light shielding pipe 6 is attached to the apparatus body 1 and the depression amount D of the light shielding pipe 6 with respect to the apparatus body 1 becomes equal to or greater than the set depression amount D1.

A switch circuit located in the control circuit 5 may also be a double switch circuit in which the first switch SW1 and third switch SW3 are connected in series. Thus, the second switch SW2 may be omitted from the above-described triple switch circuit located in the control circuit 5. In this case, power is supplied to the flashlight 2 and the flashlight 2 emits pulsed light at a stage at which the depression amount D of the light shielding pipe 6 with respect to the apparatus body 1 becomes equal to or greater than the set depression amount D1 in a state in which the light shielding pipe 6 is attached to the apparatus body 1 and the irradiation switch 15 of the apparatus body 1 is pushed.

Further, the light source unit 20 is not limited to the flashlight 2 composed of the xenon light and may be the flashlight 2 having a different structure. Alternatively, the light source unit 20 may be something that emits laser light by using a laser diode or the like.

### (Essence of the Embodiment)

The above-described embodiment is summarized below.

Thus, the optical depilation apparatus of the present embodiment is an optical depilation apparatus for performing depilation by irradiating skin with light, including: an apparatus body; a light source unit that is provided at the apparatus body and capable of emitting light for irradiating the skin; a light shielding pipe that has a skin contact surface at an end surface on a side opposite to the apparatus body in an axial direction of the light shielding pipe and is attached detachably to the apparatus body so as to surround a portion of the apparatus body which radiates light emitted from the light source unit; and a control circuit that disables the emission of light from the light source unit in a state in which the light shielding pipe is not attached to the apparatus body,
wherein:
the light shielding pipe is free to move back and forth in the axial direction;
the optical depilation apparatus further comprises a spring member that biases the light shielding pipe in the axial direction to the side opposite to the apparatus body; a plurality of contact sensors that are provided with a predetermined spacing in a circumferential direction at the skin contact surface; and an irradiation switch that is provided at the apparatus body; and
the control circuit has a first switch, a second switch and a third switch, wherein the first switch is switched OFF when the light shielding pipe is not attached to the apparatus body and when the depression amount of the light shielding pipe in the axial direction with respect to the apparatus body is less than a set depression amount that has been set in advance even when the light shielding pipe is attached to the apparatus body, wherein the first switch is ON when the light shielding pipe is attached to the apparatus body and the depression amount of the light shielding pipe with respect to the apparatus body is equal to or greater than the set depression amount, wherein the second switch is turned ON when all the contact sensors detect contact with the skin, wherein the third switch is turned ON when a user pushes the irradiation switch, wherein the control circuit interrupts power supply to the light source unit so as to disable the emission of light from the light source unit when the first switch, the second switch and the third switch is OFF, wherein the control circuit supplies power to the light source unit when the first switch, the second switch and the third switch are all turned ON, and wherein an operation port is formed at the light shielding pipe so as to expose the irradiation switch when the depression amount of the light shielding pipe with respect to the apparatus body is equal to or greater than the set depression amount.

With such a configuration, the skin surrounded by the light shielding pipe can be irradiated with light for depilation that is emitted from the light source unit in a state in which the skin contact surface of the light shielding pipe is in contact with the skin. In this case, the light irradiated from the apparatus body can be prevented from leaking to the outside by the light shielding body surrounding the portion of the apparatus body which radiates light emitted from the light source unit, and the light can be prevented from entering the eyes of the user or other people. Furthermore, with this configuration, because the light shielding pipe is detachably attached to the apparatus body, when maintenance of the apparatus body is performed, the light shielding pipe can be detached from the apparatus body, thereby facilitating the maintenance of the apparatus body. In addition, in this configuration, because the control circuit disables light emission from the light source unit in a state in which the light shielding pipe is not attached to the apparatus body, the unintended light emission can be prevented and the light can be prevented from entering the eyes of the user or other people during maintenance in which the light shielding pipe is detached from the apparatus body. Therefore, such a configuration makes it possible to obtain a highly safe optical depilation apparatus in which the light used to irradiate the skin during depilation can be prevented from leaking and entering the eyes, maintenance can be facilitated, and the light for depilation can be prevented from being emitted unintentionally and entering the eyes.

Further, the light source unit cannot emit light unless all the contact sensors provided with a predetermined spacing in a circumferential direction at the skin contact surface detect contact with the skin. Therefore, it is possible to prevent the light from leaking to the outside through a gap that is formed between the skin contact surface and the skin, when the optical depilation apparatus is used. Furthermore, with such a configuration, the light source unit can emit light only when the depression amount of the light shielding pipe with respect to the apparatus body is equal to or greater than a set depression amount and additionally when all the contact sensors provided at the skin contact surface of the light shielding pipe detect contact with the skin. Therefore, unintentional irradiation with light can be prevented even more reliably. As a result, a more safe optical depilation apparatus can be obtained.

Further, the light source unit cannot emit light unless the user pushes the irradiation switch. Therefore, unintentional emission of light can be prevented even more reliably. Furthermore, with such a configuration, the light source unit can emit light only when the depression amount of the light shielding pipe with respect to the apparatus body is equal to or greater than a set depression amount, all the contact sensors provided at the skin contact surface of the light shielding pipe detect contact with the skin, and additionally the user has pushed the irradiation switch. Therefore, an even safer optical depilation apparatus can be obtained.

## Claims

1. An optical depilation apparatus for performing depilation by irradiating skin (S) with light, comprising:
an apparatus body (1);
a light source unit (20) that is provided at the apparatus body and capable of emitting light for irradiating skin;
a light shielding pipe (6) that has a skin contact surface (11) at an end surface on a side opposite to the apparatus body in an axial direction of the light shielding pipe and is attached detachably to the apparatus body so as to surround a portion of the apparatus body which radiates light emitted from the light source unit; and
a control circuit (5) that disables the emission of light from the light source unit in a state in which the light shielding pipe is not attached to the apparatus body,
wherein:
the light shielding pipe is free to move back and forth in the axial direction;
the optical depilation apparatus further comprises a spring member (10) that biases the light shielding pipe in the axial direction to the side opposite to the apparatus body; and an irradiation switch (15) that is provided at the apparatus body, wherein
the control circuit has a first switch (SW1) and a second switch (SW3), wherein the first switch is switched OFF when the light shielding pipe is not attached to the apparatus body and when the depression amount of the light shielding pipe in the axial direction with respect to the apparatus body is less than a set depression amount that has been set in advance even when the light shielding pipe is attached to the apparatus body, wherein the first switch is ON when the light shielding pipe is attached to the apparatus body and a depression amount of the light shielding pipe with respect to the apparatus body is equal to or greater than the set depression amount, wherein the second switch is turned ON when a user pushes the irradiation switch, wherein the control circuit interrupts power supply to the light source unit so as to disable the emission of light from the light source unit when the first switch or the second switch is turned OFF, wherein the control circuit supplies power to the light source unit when the first switch is turned ON and the second switch is turned ON, and wherein an operation port (16) is formed at the light shielding pipe so as to expose the irradiation switch when the depression amount of the light shielding pipe with respect to the apparatus body is equal to or greater than the set depression amount.

2. An optical depilation apparatus according to claim 1 and further comprising a plurality of contact sensors (12) that are provided with a predetermined spacing in a circumferential direction at the skin contact surface; wherein
the control circuit has a third switch (SW2), wherein the third switch is turned ON when all the contact sensors detect contact with the skin, wherein the control circuit interrupts power supply to the light source unit so as to disable the emission of light from the light source unit when the first switch, the second switch or the third switch is OFF, and wherein the control circuit supplies power to the light source unit when the first switch, the second switch and the third switch are all turned ON.

## Patentansprüche

1. Optische Depilationsvorrichtung zum Durchführen einer Depilation durch Bestrahlen von Haut (S) mit Licht, umfassend:
einen Vorrichtungskörper (1);
eine Lichtquelleneinheit (20), die an dem Vorrichtungskörper vorgesehen ist und in der Lage ist, Licht zum Bestrahlen von Haut auszusenden;
ein Lichtabschirmrohr (6), das eine Hautkontaktfläche bzw. -oberfläche (11) an einer Endfläche bzw. -oberfläche auf einer Seite gegenüberliegend bzw. entgegensetzt zu dem Vorrichtungskörper in einer axialen Richtung des Lichtabschirmrohrs aufweist und entfernbar an dem Vorrichtungskörper angebracht ist, um einen Abschnitt des Vorrichtungskörpers zu umgeben, der Licht ausstrahlt, das von der Lichtquelleneinheit ausgesendet wird; und
eine Steuer- bzw. Regelschaltung (5), welche die Aussendung von Licht von der Lichtquelleneinheit in einem Zustand abschaltet bzw. deaktiviert, in dem das Lichtabschirmrohr nicht an dem Vorrichtungskörper angebracht ist,
wobei:
das Lichtabschirmrohr frei ist, sich in der axialen Richtung vor und zurück zu bewegen;
die optische Depilationsvorrichtung ferner ein Federglied (10), welches das Lichtabschirmrohr in der axialen Richtung zu der Seite gegenüberliegend bzw. entgegensetzt zu dem Vorrichtungskörper vorspannt; und einen Bestrahlungsschalter (15) umfasst, der an dem Vorrichtungskörper vorgesehen ist, wobei
die Steuer- bzw. Regelschaltung einen ersten Schalter (SW1) und einen zweiten Schalter (SW3) aufweist, wobei der erste Schalter AUSgeschaltet ist, wenn das Lichtabschirmrohr nicht an dem Vorrichtungskörper angebracht ist und wenn der Vertiefungs- bzw. Niederdrückbetrag des Lichtabschirmrohrs in der axialen Richtung bezüglich des Vorrichtungskörpers geringer ist als ein festgelegter Vertiefungs- bzw. Niederdrückbetrag, der im Vorhinein festgelegt wurde, selbst wenn das Lichtabschirmrohr an dem Vorrichtungskörper angebracht ist, wobei der erste Schalter EIN ist, wenn das Lichtabschirmrohr an dem Vorrichtungskörper angebracht ist und ein Vertiefungs- bzw. Niederdrückbetrag des Lichtabschirmrohrs bezüglich des Vorrichtungskörpers gleich oder größer ist als der festgelegte Vertiefungs- bzw. Niederdrückbetrag, wobei der zweite Schalter EINgeschaltet ist, wenn ein Benutzer den Bestrahlungsschalter drückt, wobei die Steuer- bzw. Regelschaltung die Leistungsversorgung zu der Lichtquelleneinheit unterbricht, um die Aussendung von Licht von der Lichtquelleneinheit abzuschalten bzw. zu deaktivieren, wenn der erste Schalter oder der zweite Schalter Ausgeschaltet ist, wobei die Steuer- bzw. Regelschaltung der Lichtquelleneinheit Leistung zuführt, wenn der erste Schalter EINgeschaltet ist und der zweite Schalter EINgeschaltet ist, und wobei eine Betriebsöffnung (16) an dem Lichtabschirmrohr gebildet ist, um den Bestrahlungsschalter freizulegen, wenn der Vertiefungs- bzw. Niederdrückbetrag des Lichtabschirmrohrs bezüglich des Vorrichtungskörpers gleich oder größer ist als der festgelegte Vertiefungs- bzw. Niederdrückbetrag.

2. Optische Depilationsvorrichtung nach Anspruch 1 und ferner umfassend eine Mehrzahl von Kontaktsensoren (12), die mit einem vorbestimmten Abstand in einer Umfangsrichtung an der Hautkontaktfläche vorgesehen sind; wobei
die Steuer- bzw. Regelschaltung einen dritten Schalter (SW2) aufweist, wobei der dritte Schalter EINgeschaltet ist, wenn alle die Kontaktsensoren einen Kontakt mit der Haut detektieren, wobei die Steuer- bzw. Regelschaltung eine Leistungsversorgung zu der Lichtquelleneinheit unterbricht, um die Aussendung von Licht von der Lichtquelleneinheit abzuschalten bzw. zu deaktivieren, wenn der erste Schalter, der zweite Schalter oder der dritte Schalter AUS ist, und wobei die Steuer- bzw. Regelschaltung der Lichtquelleneinheit Leistung zuführt, wenn der erste Schalter, der zweite Schalter und der dritte Schalter alle EINgeschaltet sind.

## Revendications

1. Appareil de dépilation optique pour réaliser une dépilation en irradiant la peau (S) de lumière, comprenant :
un corps d'appareil (1) ;
un module de source lumineuse (20) qui est prévu au niveau du corps d'appareil et capable d'émettre de la lumière pour irradier la peau ;
un tuyau de protection lumineuse (6) qui possède une surface de contact cutané (11) au niveau d'une surface d'extrémité sur un côté opposé au corps d'appareil dans une direction axiale du tuyau de protection lumineuse et est relié de manière amovible au corps d'appareil de manière à entourer une portion du corps d'appareil qui rayonne la lumière émise depuis le module de source lumineuse ; et
un circuit de commande (5) qui désactive l'émission de lumière depuis le module de source lumineuse dans un état dans lequel le tuyau de protection lumineuse n'est pas relié au corps d'appareil,
dans lequel :
le tuyau de protection lumineuse est libre de se déplacer en va-et-vient dans la direction axiale ;
l'appareil de dépilation optique comprend en outre un élément de ressort (10) qui incline le tuyau de protection lumineuse dans la direction axiale vers le côté opposé au corps d'appareil ; et un commutateur d'irradiation (15) qui est prévu au niveau du corps d'appareil, dans lequel
le circuit de commande possède un premier commutateur (SW1) et un deuxième commutateur (SW3), dans lequel le premier commutateur est éteint lorsque le tuyau de protection lumineuse n'est pas relié au corps d'appareil et lorsque la quantité d'enfoncement du tuyau de protection lumineuse dans la direction axiale par rapport au corps d'appareil est inférieure à une quantité d'enfoncement définie qui a été définie à l'avance même lorsque le tuyau de protection lumineuse est relié au corps d'appareil, dans lequel le premier commutateur est allumé lorsque le tuyau de protection lumineuse est relié au corps d'appareil et une quantité d'enfoncement du tuyau de protection lumineuse par rapport au corps d'appareil est supérieure ou égale à la quantité d'enfoncement définie, dans lequel le deuxième commutateur est allumé lorsqu'un utilisateur appuie sur le commutateur d'irradiation, dans lequel le circuit de commande interrompt l'alimentation électrique vers le module de source lumineuse de manière à désactiver l'émission de lumière depuis le module de source lumineuse lorsque le premier commutateur ou le deuxième commutateur est éteint, dans lequel le circuit de commande alimente électriquement le module de source lumineuse lorsque le premier commutateur est allumé et le deuxième commutateur est allumé, et dans lequel un port de fonctionnement (16) est formé au niveau du tuyau de protection lumineuse de manière à exposer le commutateur d'irradiation lorsque la quantité d'enfoncement du tuyau de protection lumineuse par rapport au corps d'appareil est supérieure ou égale à la quantité d'enfoncement définie.

2. Appareil de dépilation optique selon la revendication 1 et comprenant en outre une pluralité de capteurs de contact (12) qui sont prévus avec un espacement prédéterminé dans une direction périphérique au niveau de la surface de contact cutané ; dans lequel
le circuit de commande possède un troisième commutateur (SW2), dans lequel le troisième commutateur est allumé lorsque l'ensemble des capteurs de contact détectent un contact avec la peau, dans lequel le circuit de commande interrompt l'alimentation électrique vers le module de source lumineuse de manière à désactiver l'émission de lumière depuis le module de source lumineuse lorsque le premier commutateur, le second commutateur ou le troisième commutateur est éteint, et dans lequel le circuit de commande alimente électriquement le module de source lumineuse lorsque le premier commutateur, le second commutateur et le troisième commutateur sont tous allumés.
